# EUROPÄISCHE PATENTSCHRIFT

(11) **EP 3 534 734 B1**
(45) Veröffentlichungstag und Bekanntmachung des Hinweises auf die Patenterteilung: **23.09.2026**
(21) Anmeldenummer: 17798124.8
(22) Anmeldetag: 26.10.2017
(51) Int. Cl.: A24F 40/46, A24F 40/485, A24F 40/50, A61M 15/06, A61M 11/00, A24F 40/10

(54) **VERDAMPFEREINHEIT FÜR EINEN INHALATOR**
VAPORISER UNIT FOR AN INHALER
UNITÉ DE VAPORISATION POUR UN INHALATEUR

(30) Priorität: 01.11.2016 DE 102016120803
(43) Veröffentlichungstag der Anmeldung: 11.09.2019
(73) Patentinhaber: Körber Technologies GmbH, 21033 Hamburg (DE)
(72) Erfinder: SCHMIDT, Rene, 21244 Buchholz i.d.N. (DE); KESSLER, Marc, 22415 Hamburg (DE); KALAYDZHYAN, Karen, 22607 Hamburg (DE); TRIEU, Hoc Khiem, 21394 Westergellersen (DE); BOHNE, Sven, 22297 Hamburg (DE)
(74) Vertreter: D Young & Co LLP
(86) Internationale Anmeldenummer: PCT/EP2017/077459
(87) Internationale Veröffentlichungsnummer: WO 2018/083007

(56) Entgegenhaltungen:
- EP-A1- 2 399 636
- EP-A1- 3 078 281
- WO-A1-2015/117704
- US-A1- 2014 238 424
- US-A1- 2015 059 780

## Beschreibung

Die vorliegende Erfindung betrifft eine Verdampfereinheit für einen Inhalator, mit einem Verdampfungskörper, welcher eine Einlassseite, eine Auslassseite, eine Mehrzahl von Mikrokanälen, ein Substrat und ein Widerstandsheizelement zum Verdampfen von durch die Mikrokanäle geförderter Flüssigkeit aufweist.

Aktuelle auf dem Docht-Wendel-Prinzip aufbauende elektronische Zigaretten, wie beispielhaft in der US 2016/0021930 A1 beschrieben, weisen verschiedene Nachteile auf. Erstens sind Liquidverdampfung und -dosierung nicht voneinander getrennt. Zweitens stehen Dampfmenge und Heizertemperatur in einem direkten Zusammenhang, d.h. hohe Dampfmengen bedingen hohe Heizleistungen bzw. Verdampfertemperaturen. Drittens kommt es zu ungleichmäßigen Temperaturbereichen am/im Bereich des Verdampfers mit der Gefahr lokaler Liquid-Überhitzung und Schadstoffentstehung. Viertens weicht die im Verdampferbereich an der Docht-/Wendeloberfläche anliegende Temperatur von der Kerntemperatur im Docht ab, wodurch es zu Konzentrationsänderungen der Liquidbestandteile während eines jeden einzelnen Verdampfungsvorgangs bzw. Puffs oder Zugs kommt. Diese Konzentrationsänderung führt darüber hinaus zu einer allmählichen Veränderung der Zusammensetzung des noch im Flüssigkeitsspeicher befindlichen Liquids, d.h. auch die freigesetzte Wirkstoffmenge ist nicht gleichmäßig und ändert sich von Zug zu Zug.

Die aus der US 2016/0262454 A1 bekannte Verdampfereinheit weist einen Verdampfungskörper auf, gebildet aus dem Substrat, aus in dem Substrat zu einem Teil ausgebildeten und zum anderen Teil von einem Deckel mit Mikroperforationen abgedeckten kapillaren Mikrokanälen sowie dem in dem Substrat vorgesehenen Flüssigkeitsspeicher und einem in dem Substrat vorgesehenen Verdampferraum, in dem der sich eben flächig erstreckende MEMS-basierte Heizer angeordnet ist. Die sich zwischen dem Flüssigkeitsspeicher und dem Verdampferraum erstreckenden in Dünnfilmschichtverfahren aufgebauten kapillaren Mikrokanäle sind mit Ventilen als Transportelemente versehen und parallel zu den Dünnfilmschichtlagen beziehungsweise zu der Längserstreckung des Substrats angeordnet. Im Gebrauch wird Liquid unter Kapillarkraftwirkung von dem Flüssigkeitsspeicher durch die kapillaren Mikrokanäle zu der Einlassseite des Verdampferraums gefördert, in dem Verdampferraum des MEMS-basierten Heizers verdampft und an der Auslassseite des Verdampferraums durch den Deckel mit den Mikroperforationen abgegeben. Der sich eben flächig erstreckende MEMS-basierte Heizer vermag das Liquid nur eingeschränkt zu verdampfen, wonach die hiermit erzielbare Verdampfungsleistung relativ gering ist. Der Aufbau mit dem Flüssigkeitsspeicher und dem Verdampferraum in dem Substrat ist komplex und benötigt relativ viel Bauraum. Das Abdecken der teilweise im Substrat ausgebildeten kapillaren Mikrokanäle mit dem Deckel bringt Abdichtungsprobleme mit sich, auch zwischen den kapillaren Mikrokanälen, ebenso wie einen erhöhten Fertigungsaufwand.

Die aus der US 2016/0007653A1 bekannte Verdampfereinheit ist zusammengestellt aus einer Leiterplatte mit gedruckter Schaltung, einem auf der Leiterplatte angeordneten, als MEMS-Verdampfer-Chip ausgebildeten Verdampfungskörper sowie einem Gehäuseteil, in das die den MEMS-Verdampfer-Chip tragende Leiterplatte unter Belassen eines Vorratsraums für Liquid mit einem Ende an einer Gehäusekante eingesetzt ist. Der MEMS-Verdampfer-Chip ist ausgehend von einem Siliziumsubstrat mehrschichtig in Dünnfilmschichttechnik aufgebaut, mit einer kapillaren, von einer Membran abgedeckten Mikrokanalanordnung, einem Widerstandsheizer und einem Widerstandstemperatursensor, die auf der der Mikrokanalanordnung abgewandten Seite der Membran angeordnet sind und einen Abschnitt der Mikrokanalanordnung übergreifen. Die Mikrokanäle der Mikrokanalanordnung sind mit ihrer Längsachse parallel zu den Dünnfilmschichtlagen angeordnet und zu einem Teil in dem Siliziumsubstrat und zu einem anderen Teil durch einen Deckel in Form der Membran ausgebildet. An den beiden Enden der Mikrokanalanordnung ist jeweils eine Kavität ausgebildet, wovon die eine Kavität als Verdampfungsauslassraum und die andere Kavität im Zusammenwirken mit den Innenwänden des Gehäuseteils als Vorratsraum oder als Flüssigkeitsspeicher für Liquid dient. Im Gebrauch wird Liquid unter Kapillarkraftwirkung fortlaufend in die Mikrokanalanordnung gefördert und mittels des Widerstandsheizers verdampft, wonach Liquidfördern und Liquidverdampfen nicht getrennt voneinander beeinflussbar, sondern voneinander abhängig sind, was ein genaueres Steuern oder Regeln des Verdampfens von Liquid erschwert. Der übergreifende, vom Kanalinneren und dem Liquid beabstandete außenseitig auf der Membran angeordnete Widerstandsheizer vermag das Liquid nur eingeschränkt zu verdampfen, wonach die hiermit erzielbare Verdampfungsleistung relativ gering ist. Der Aufbau ist thermisch träge und mit einer Vielzahl von Schichten komplex. Das Abdecken der teilweise im Substrat ausgebildeten, zunächst nach oben geöffnet, nutartig hergestellten Mikrokanäle mit der Membran bringt Abdichtungsprobleme mit sich, auch zwischen den Mikrokanälen, ebenso wie einen erhöhten Fertigungsaufwand.

EP 3 078 281 A1, US 2014/0238424 A1 und US 2015/0059780 A1 offenbaren jeweils eine Verdampfereinheit für eine elektronische Zigarette mit einem durch Leerräume zwischen Gewebefasern einer Gewebematte bzw. zwischen Kugelhaufen, d.h. Porenkörpern oder Partikeln, ausgeformtem Flüssigkeits-Transportgebilde.

EP 2 399 636 A1 offenbart eine weitere Verdampfeinheit für eine elektronische Zigarette.

WO 2015/117704 A1 offenbart ein Aerosol-erzeugendes System, wobei der Verdampfungskörper ein Widerstandsheizelement in Form eines Drahtnetzes (mesh) aufweist.

Die Aufgabe der Erfindung besteht darin, eine bekannte Verdampfereinheit in verschiedener Hinsicht zu verbessern, insbesondere die Verdampferleistung zu erhöhen, die Komplexität des Aufbaus zu verringern, einen geringeren Bauraum zu ermöglichen und/oder die Abdichtungsproblematik zu verringern, sowie eine einfache, zuverlässig und reproduzierbar arbeitende Verdampfereinheit für einen Inhalator bereitzustellen, welche die obigen Nachteile überwindet und in der Lage ist, eine Dampfmenge mit gewünschten Eigenschaften, wie Menge und Zusammensetzung, bereitzustellen, sowie ein entsprechendes Steuerverfahren anzugeben.

Die Erfindung löst diese Aufgabe mit den Merkmalen des unabhängigen Anspruchs. Erfindungsgemäß wird das Widerstandsheizelement von dem Substrat gebildet, so dass der Heizstrom direkt durch das Substrat selbst fließt. Im Unterschied zum Stand der Technik wird die Heizspannung an das Substrat selbst angelegt. Das erhitzte Substrat kann direkt die in den Mikrokanälen enthaltene Flüssigkeit verdampfen. Diese unmittelbare Wärmeübertragung auf die Flüssigkeit ist erheblich effektiver und schneller als die bekannte Erwärmung mittels eines separaten metallischen Heizelements.

Erfindungsgemäß sind die Mikrokanäle jeweils von dem Substrat in Umfangsrichtung umschlossen. Auf diese Weise kann ein effektives Verdampfen in kompakter Bauweise gewährleistet werden. Die Mikrokanäle können auf einfache Art und Weise gewissermaßen mit einer optimal ansteuerbaren Vollmantelheizung versehen und Abdichtungsprobleme verursachende Abdeckungen, wie sie bei bekannten Verdampfungskörpern verwendet werden, vermieden werden. Vorzugsweise entspricht die Länge eines oder jedes Mikrokanals der Dicke des Substrats.

Vorzugsweise ist auf der Einlassseite des Substrats eine den Durchfluss von Flüssigkeit durch die Mikrokanäle steuernde Durchflusssteuereinrichtung vorgesehen. Dies ermöglicht eine präzise Dosierung der zu verdampfenden Flüssigkeit in die Mikrokanäle des Verdampfers. Zudem kann eine je Zug gleichbleibende Aerosolqualität, insbesondere Dampfzusammensetzung und Tropfenspektrum, gewährleistet und eine Rückwirkung auf das Flüssigkeitsspeicher infolge ungleichmäßiger Komponentenverdampfung weitgehend verhindert werden. Die Mikrokanäle lassen sich mit einer exakt bemessenen Portion an Flüssigkeit befüllen. Die Portion kann rückflusssicher in den Mikrokanälen gehalten und zum portionsgenauen vollständigen Verdampfen bereitgestellt werden. Die Durchflusssteuereinrichtung kann beispielsweise in Form einer oder mehrere Pumpen und/oder eines oder mehrerer Ventile ausgebildet sein.

In einer vorteilhaften Ausführungsform ist die Durchflusssteuereinrichtung eine Durchflusssteuerschicht mit sich durch die Durchflusssteuerschicht erstreckenden Durchgangsbohrungen. Besonders vorteilhaft ist dabei der Kontaktwinkel zwischen Flüssigkeit und der Innenwand einer oder jeder Durchgangsbohrung durch Anlegen einer mittels einer Spannungsquelle erzeugbaren elektrischen Spannung, bzw. eines von der elektrischen Spannung erzeugten elektrischen Feldes, veränderbar. Hierdurch ist vorteilhaft der Durchfluss durch die Durchgangsbohrungen veränderbar, insbesondere stoppbar und/oder, insbesondere durch ein Verringern oder ein Abschalten der elektrischen Spannung, freigebbar. Auf diese Weise lässt sich ein exaktes Fördern und Bemessenen einer Portion an Flüssigkeit in den Mikrokanälen sowie eine Rückflusssicherung und ein portionsgenaues und vollständiges Verdampfen realisieren. Hierbei wird vorzugsweise der Effekt der elektrischen Benetzung (electro wetting) genutzt.

Vorteilhaft ist zwischen der Durchflusssteuerschicht und dem Substrat eine Isolierschicht mit sich durch die Isolierschicht erstreckenden Durchgangsöffnungen vorgesehen. Hierdurch kann eine ungewollte Verdampfung von Flüssigkeit in dem Flüssigkeitsspeicher bzw. in der Durchflusssteuerschicht und den dortigen Durchflussbohrungen während der Verdampfung wirksam verhindert werden. Ebenso wird ein unerwünschter Wärmetransfer in den Flüssigkeitsspeicher vermieden. Mit anderen Worten lässt sich der Wärmeeintrag in das Substrat optimieren und ein unerwünschtes Erwärmen oder gar Verdampfen in dem Substrat vorgelagerter, Flüssigkeit enthaltender Abschnitte zuverlässig vermeiden.

Besonders vorteilhaft ist in einem Datenspeicher eine dem verwendeten Flüssigkeitsgemisch angepasste Spannungskurve Uh(t) hinterlegt. Der Heizspannungsverlauf Uh(t) ist dem verwendeten Flüssigkeitsgemisch angepasst vorgegeben und die Heiztemperatur des Substrats kann gemäß der exakt bekannten Verdampfungskinetik des jeweiligen Flüssigkeitsgemischs zeitlich über den Verdampfungsvorgang gesteuert werden. Auf diese Weise lässt sich ein optimales Verdampfen der Flüssigkeit angepasst an deren Komponenten gewährleisten und ein Entstehen unerwünschter Zersetzungsprodukte zuverlässig vermeiden.

Die Heiztemperatur kann zeitlich über den Verdampfungsvorgang hochfrequent gesteuert oder geregelt werden.

Nach einem weiteren Aspekt der Erfindung wird ein Verfahren zum Steuern einer zuvor beschriebenen Verdampfereinheit bereitgestellt. Vorteilhaft wird dabei der Heizspannungsverlauf Uh(t) dem verwendeten Flüssigkeitsgemisch angepasst vorgegeben und somit die Heiztemperatur des Substrats gemäß der Verdampfungskinetik des jeweiligen Flüssigkeitsgemischs zeitlich über den Verdampfungsvorgang gesteuert. Auf diese Weise lässt sich ein optimales Verdampfen der Flüssigkeit, angepasst an deren Komponenten gewährleisten und ein Entstehen unerwünschter Zersetzungsprodukte zuverlässig vermeiden. Mit einem derart ausgestalteten Verdampfen kann der Wärmeeintrag von dem Substrat in die Flüssigkeit optimiert werden. Ein Aufbauen von Grenzschichten zwischen Flüssigkeit und Wandungen der Mikrokanäle in Form von Verdampfungsblasen, welche den Wärmeübergang behindern können oder die Gefahr einer lokalen Überhitzung darstellen, kann zuverlässig verringert werden.

Bei dem für bekannte Verdampfungskörper bekannten Betrieb mit einer über das Verdampfen hin einmalig eingestellten konstanten Temperatur ist die Temperatur auf ein Verdampfen der höchstsiedende Komponente konstant vorgegeben und eingestellt, um ein vollständiges Verdampfen der Flüssigkeit zu gewährleisten, da man davon ausgeht, dass leichter siedende Komponenten bei Anlegen der Maximaltemperatur ohnehin Verdampfen. Die bekannte Betriebsweise birgt die Gefahr in sich, dass jede niedriger siedende Komponente der aus mehreren Komponenten bestehenden Flüssigkeit Verdampfungsblasen und damit eine Grenzschicht zwischen Flüssigkeit und Wandungen der Mikrokanäle ausbildet und somit den Wärmeeintrag behindern kann. In Abkehr von dieser bekannten Weise die Temperatur einer Verdampfereinheit zu steuern kann mit dem vorgeschlagenen Verfahren, insbesondere in seinen weiteren Ausgestaltungen, ein Minimieren einer Grenzschichtbildung und ein optimaler Wärmeeintrag erreicht werden.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorzugsweise erfolgt das Verdampfen einer Portion an Flüssigkeit, welche in den Verdampfungskörper 60 gefördert ist, gestuft mit einer Ansteuerfrequenz des Heizens des Substrats 63 im Bereich von 1 Hz bis 50 kHz, bevorzugt im Bereich von 30 Hz bis 30 kHz, besonders bevorzugt im Bereich von 100 Hz bis 25 kHz.

Die Erfindung wird im Folgenden anhand bevorzugter Ausführungsformen unter Bezugnahme auf die beigefügten Figuren erläutert. Dabei zeigt
- Fig. 1: eine Querschnittsansicht eines elektronischen Zigarettenprodukts in einer Ausführungsform der Erfindung;
- Fig. 2: eine Querschnittsansicht einer Kartusche für ein elektronisches Zigarettenprodukt;
- Fig. 3: eine perspektivische Schnittansicht einer Verdampfereinheit; und
- Fig. 4A-4E: die Verdampfereinheit gemäß Figur 3 in unterschiedlichen Zuständen des Verdampfungsprozesses.

Das elektronische Zigarettenprodukt 10 umfasst ein im Wesentlichen stabförmiges oder zylindrisches Gehäuse 11. In dem Gehäuse 11 ist ein Luftkanal 30 zwischen mindestens einer Lufteinlassöffnung 31 und dem Mundende 32 des Zigarettenprodukts 10 vorgesehen. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt. Mindestens eine Lufteinlassöffnung 31 kann an der Mantelseite des Gehäuses 11 angeordnet sein. Zusätzlich oder alternativ kann mindestens eine Lufteinlassöffnung 31A am entfernten Ende 33 des Zigarettenprodukts 10 angeordnet sein. Das entfernte Ende 33 des Zigarettenprodukts 10 bezeichnet das dem Mundende 32 entgegengesetzte Ende des Zigarettenprodukts 10.

Nach einer oder beiden Lufteinlässen 31, 31A kann im Strömungsweg der Luftströmung 34 vorteilhaft eine Lufterwärmungseinrichtung 37 zum Erwärmen bzw. Vorwärmen der eintretenden Luft angeordnet sein. Hierdurch kann die Aerosolbildung optimiert werden. Die Lufterwärmungseinrichtung 37 kann beispielsweise benachbart zu der Energieversorgungseinheit 14 angeordnet sein und/oder sich in Umfangsrichtung um die Mantelinnenseite des Gehäuses 11 erstrecken.

Die durch die Einlassöffnung 31 angesaugte Luft wird in dem Luftkanal 30, ggf. über die Schnittstelle bzw. Trennfläche 57 zu einer Verdampfereinheit 20 geleitet. Die Verdampfereinheit 20 gibt Flüssigkeit 50 aus dem Flüssigkeitsspeicher 18 als Zugabe 40 in Form kleiner Flüssigkeitstropfen als Nebel/Aerosol und/oder gasförmig als Dampf in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

Das Zigarettenprodukt 10 umfasst, vorteilhaft am entfernten Ende 33 des Zigarettenprodukts 10, eine elektronische Energieversorgungseinheit 12 mit einem elektrischen Energiespeicher 14 und einer elektrischen/elektronischen Einheit 15. Der Energiespeicher 14 kann insbesondere eine elektrochemische Einweg-Batterie oder ein wiederaufladbarer elektrochemischer Akku, z.B. ein Li-lonen-Akku, sein. Das Zigarettenprodukt 10 umfasst des Weiteren, vorteilhaft am Mundende 32 des Zigarettenprodukts 10, eine Verbrauchseinheit 17 mit einem Flüssigkeitsspeicher 18, einer elektrischen Einheit 19 und der Verdampfereinheit 20.

Anstelle der getrennten elektrischen/elektronischen Einheiten 15, 19 kann auch eine einheitliche elektrische/elektronische Einheit vorgesehen sein, die entweder in der Energieversorgungseinheit 12 oder in der Verbrauchseinheit 17 angeordnet sein kann. Die Gesamtheit der elektrischen/elektronischen Einheiten des Zigarettenprodukts 10 wird im Folgenden als Steueranordnung 29 bezeichnet.

In dem Gehäuse 11 ist vorteilhaft ein Sensor, beispielsweise ein Drucksensor oder ein Druck- oder Strömungsschalter, angeordnet, wobei die Steueranordnung auf der Grundlage eines von dem Sensor ausgegebenen Sensorsignals einen Betriebszustand des Zigarettenprodukts 10, in dem ein Konsument am Mundende 32 des Zigarettenprodukts 10 zieht, um zu inhalieren, feststellen kann. In diesem Betriebszustand steuert die Steueranordnung 29 die Verdampfereinheit 20 an, um Flüssigkeit 50 aus dem Flüssigkeitsspeicher 18 als Zugabe 40 in Form kleiner Flüssigkeitstropfen als Nebel/Aerosol und/oder gasförmig als Dampf in den Luftstrom 34 zuzugeben.

Die in dem Flüssigkeitsspeicher 18 gespeicherte, zu dosierende Flüssigkeit (d.h. das flüssige Komponentengemisch) ist beispielsweise eine Mischung aus 1,2-Propylenglykol, Glycerin und/oder Wasser, der ein oder mehrere Aromen (Flavour) und/oder Wirkstoffe, wie beispielsweise Nikotin, zugemischt sein können.

Die Verbrauchseinheit 17 ist vorteilhaft als vom Konsumenten auswechselbare Kartusche 21, d.h. als Einwegteil ausgeführt. Der insbesondere den Energiespeicher 14 enthaltende Rest des Zigarettenprodukts 10 ist vorteilhaft als vom Konsumenten wiederverwendbares Grundteil 56, d.h. als Mehrwegteil ausgeführt. Die Kartusche 21 ist vom Konsumenten mit dem Grundteil 56 verbindbar und vom Grundteil 56 lösbar ausgebildet. Zwischen der Kartusche 21 und dem wiederverwendbaren Grundteil 56 ist somit eine Trennfläche bzw. Schnittstelle 57 gebildet. Das Kartuschengehäuse 58 kann einen Teil des Gehäuses 11 des Zigarettenprodukts 10 bilden.

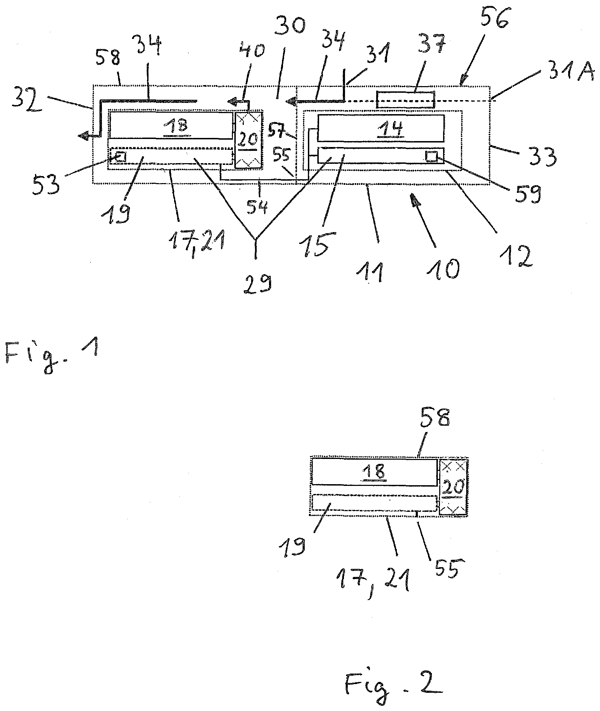

In anderen Ausführungsformen, siehe Fig. 2, ist die Verbrauchseinheit 17 als Kartusche 21 ausgeführt, die in den wiederverwendbaren Grundteil 56 des Zigarettenprodukts 10 durch den Konsumenten einsetzbar und aus diesem entnehmbar ist. Das Kartuschengehäuse 58 ist in diesem Fall ein von dem Gehäuse 11 des Zigarettenprodukts 10 separates Gehäuse.

Die Kartusche 21 umfasst mindestens den Flüssigkeitsspeicher 18. Die Kartusche 21 kann, wie in Fig. 2 gezeigt, die elektrische/elektronische Einheit 19 umfassen. In anderen Ausführungsformen ist die elektrische/elektronische Einheit 19 ganz oder teilweise fester Bestandteil des Grundteils 56. Ebenso kann die Verdampfereinheit 20 Teil der Kartusche 21 oder in dem Grundteil 56 angeordnet sein. Die Kartusche 21 kann daher in manchen Ausführungsformen im Wesentlichen nur aus dem Flüssigkeitsspeicher 18 bestehen und ggf. dem Kartuschengehäuse 58, wobei das Kartuschengehäuse 58 alternativ von dem Gehäuse des Flüssigkeitsspeichers 18 gebildet sein kann, so dass ein separates Kartuschengehäuse 58 entbehrlich sein kann.

Die Kartusche 21 kann neben der Verwendung in stabförmigen Zigarettenprodukten 10 auch in anderen Inhalatoren eingesetzt werden, beispielsweise in einer elektronischen Pfeife, Shisha, anderen Heat-not-burn-Produkten, oder einem medizinischen Inhalator. Der Energiespeicher 14 ist in der Regel nicht Teil der Kartusche 21, sondern Teil des wiederverwendbaren Grundteils 56.

Die Verbrauchseinheit 17 bzw. die Kartusche 21 umfasst vorteilhaft einen nichtflüchtigen Informationsspeicher 53 (siehe Fig. 1) zum Speichern von die Verbrauchseinheit 17 bzw. die Kartusche 21 betreffender Information bzw. Parametern, beispielsweise in Ausführung als EEPROM, RFID oder anderer geeigneter Form. Der Informationsspeicher 53 kann Teil der elektrischen/elektronischen Einheit 19 oder separat davon ausgebildet sein. In dem Informationsspeicher 53 gespeichert ist vorteilhaft Information zum Inhaltsstoff, d.h. zur Zusammensetzung der in dem Flüssigkeitsspeicher 18 gespeicherten Flüssigkeit; Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, insbesondere umfassend eine ID zur eindeutigen Kennzeichnung der Verbrauchseinheit 17 bzw. Kartusche 21; Seriennummer, Herstelldatum und/oder Ablaufdatum; und/oder Zugzahl (Anzahl der Inhalationszüge durch den Konsumenten) bzw. der Nutzungszeit. Der Datenspeicher 53 ist vorteilhaft über Kontakte und/oder Leitungen mit der Steuereinrichtung 15 des Grundteils 56 verbunden oder verbindbar.

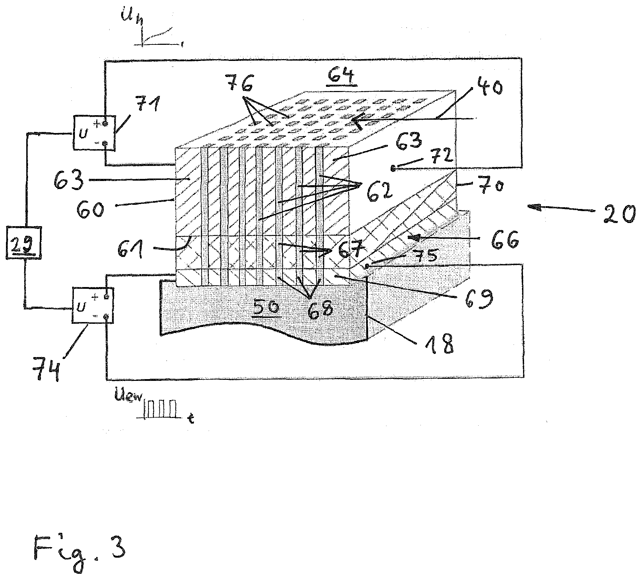

Eine vorteilhafte Ausführungsform einer erfindungsgemäßen Verdampfereinheit 20 ist in Fig. 3 gezeigt. Die Verdampfereinheit 20 umfasst einen Verdampfungskörper 60 mit einem blockförmigen Substrat 63 aus einem elektrisch leitenden Material, vorzugsweise Silizium, dotierte Keramik, Metall-Keramik, Filter-Keramik, Halbleiter, insbesondere Germanium, Graphit, Halbmetall und/oder Metall. Das Substrat 63 ist mit einer Mehrzahl von Mikrokanälen 62 versehen, die eine Einlassseite 61 des Substrats 63 mit einer Auslassseite 64 flüssigkeitsleitend verbinden. Die Einlassseite 61 ist flüssigkeitsleitend mit dem Flüssigkeitsspeicher 18 verbunden. Dies wird im Folgenden noch genauer erläutert.

Der mittlere Durchmesser der Mikrokanäle 62 liegt vorzugsweise im Bereich zwischen 5 µm und 100 µm, weiter vorzugsweise im Bereich zwischen 10 µm und 50 µm, noch weiter vorzugsweise im Bereich zwischen 20 µm und 40 µm und beträgt beispielsweise 30 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben steigt, bis der Mikrokanal 62 mit Flüssigkeit gefüllt ist.

Die Anzahl der Mikrokanäle 62 liegt vorzugsweise im Bereich zwischen vier und 100. Auf diese Weise lässt sich der Wärmeeintrag von dem Substrat in die Mikrokanäle 62 optimieren und eine gesicherte hohe Verdampfungsleistung sowie eine ausreichend große Dampfaustrittsfläche realisieren.

Die Mikrokanäle 62 sind in Form eines quadratischen, rechteckigen, vieleckigen, runden, ovalen oder anders geformten Arrays angeordnet, wie in Figur 3 ersichtlich ist. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 20 und/oder z vorteilhaft im Bereich zwischen 2 und 50 und weiter vorteilhaft im Bereich zwischen 3 und 20 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 62 liegt vorzugsweise im Bereich zwischen 100 µm und 500 µm, weiter vorzugsweise im Bereich zwischen 150 µm und 400 µm noch weiter vorzugsweise im Bereich zwischen 180 µm und 370 µm und beträgt beispielsweise 300 µm. Auf diese Weise lässt sich eine optimale Flüssigkeitsaufnahme und Portionsbildung bei ausreichend gutem Wärmeeintrag von dem Substrat 63 in die Mikrokanäle 62 realisieren.

Der Abstand zweier Mikrokanäle 62 beträgt vorzugsweise mindestens das 1,3-fache des hydraulischen Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des hydraulischen Durchmessers eines Mikrokanals 62 betragen. Auf diese Weise lässt sich ein optimaler Wärmeeintrag von dem Substrat in die Mikrokanäle und eine ausreichend stabile Anordnung und Wandstärke der Mikrokanäle realisieren.

Die Verdampfereinheit 20 weist eine vorzugsweise von der Steuereinheit 19 steuerbare Heizspannungsquelle 71 auf, die über Elektroden 72 an gegenüberliegenden Seiten des Substrats 63 mit diesem verbunden ist, so dass eine von der Heizspannungsquelle 71 erzeugte elektrische Spannung Uh zu einem Stromfluss durch das Substrat 63 führt. Aufgrund des Ohmschen Widerstands des Substrats 63 führt der Stromfluss zu einer Erhitzung des Substrats 63 und daher zu einer Verdampfung von in den Mikrokanälen 62 enthaltener Flüssigkeit. Der auf diese Weise erzeugte Dampf und/oder Aerosol entweicht zur Auslassseite 64 aus den Mikrokanälen 62 und wird als Dampfzugabe 40 der Luftströmung 34 beigemischt, siehe Figur 1. Genauer steuert bei Feststellung eines durch Ziehen des Konsumenten verursachten Luftstroms 34 durch den Luftkanal 30 die Steueranordnung 29 die Heizspannungsquelle 71 an, wobei durch spontane Erhitzung die in den Mikrokanälen 62 befindliche Flüssigkeit in Form von Dampf und/oder Aerosol 40 aus den Mikrokanälen 62 getrieben wird. Der erzeugte Dampf bzw. das Aerosol 40 wird der optional vorgewärmten Luftströmung 34 zugeführt, indem diese außen an den Austrittsöffnungen 76 des Verdampfungskörpers 60 vorbeiströmt, siehe Figuren 1 und 3. Vorzugsweise ist in dem Datenspeicher 53 der Kartusche 21 oder in einem Datenspeicher 59 des Grundteils 56 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Blocks bzw. Substrats 63, und damit auch die Temperatur der kapillaren Mikrokanäle 62, gemäß der exakt bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100 °C und 400 °C, weiter bevorzugt zwischen 150°C und 350 °C, noch weiter bevorzugt zwischen 190 °C und 290 °C.

An der Einlassseite 61 des Substrats 60 ist eine den Durchfluss von Flüssigkeit durch die Mikrokanäle 62 steuernde Durchflusssteuereinrichtung 66 vorgesehen ist. Die Durchflusssteuereinrichtung 66 ist in einer vorteilhaften Ausführungsform eine Durchflusssteuerschicht 69 mit sich durch die Durchflusssteuerschicht 69 erstreckenden Durchgangsbohrungen 68.

Besonders vorteilhaft ist in der Durchflusssteuerschicht 66 ein elektrisches Feld an die Flüssigkeit zum Beeinflussen des Kontaktwinkels zwischen Flüssigkeit und Innenwand einer Durchgangsbohrung 68 anlegbar, wobei vorzugsweise der Effekt der elektrischen Benetzung (electro wetting) genutzt wird. Zu diesem Zweck weist die Verdampfereinheit 20 eine vorzugsweise von der Steuereinheit 19 steuerbare EW (electro wetting)-Spannungsquelle 74 auf, die über Elektroden 75 an gegenüberliegenden Seiten der Durchflusssteuerschicht 69 mit diesem verbunden ist, so dass eine von der EW-Spannungsquelle 74 erzeugte elektrische Spannung Uew zu einer Verschiebung der Ladungsträger in der Flüssigkeit in den Durchgangsbohrungen 68 führt. Auf diese Weise kann der Kontaktwinkel zwischen Flüssigkeit und Innenwand einer Durchgangsbohrung 68 zwischen hydrophil (Anlegen einer geeigneten Spannung) und hydrophob (keine Spannung) geändert werden. Wenn der Kontaktwinkel in den Durchgangsbohrungen 68 auf hydrophil eingestellt ist, wird die Flüssigkeit aus dem Flüssigkeitsspeicher 18 kapillar in die Durchgangsbohrungen 68 gefördert und kann aufgrund der kapillaren Förderwirkung in die Durchgangsöffnungen 67 und weiter in die Mikrokanäle 62 steigen. Wenn der Kontaktwinkel in den Durchgangsbohrungen 68 auf hydrophob eingestellt ist, ist ein Aufsteigen der Flüssigkeit aus dem Flüssigkeitsspeicher 18 durch die Durchgangsbohrungen 68 in die Durchgangsöffnungen 67 und in die Mikrokanäle 62 gesperrt. Da keine kapillare Förderwirkung auftritt, verbleibt die Flüssigkeit im Flüssigkeitsspeicher 18. Die Funktion der Durchflusssteuerschicht 69 besteht also darin, ein Umschalten zwischen freiem Flüssigkeitsdurchtritt durch die Durchgangsbohrungen 68 und Sperren von Flüssigkeitsdurchtritt durch die Durchgangsbohrungen 68 zu ermöglichen. Die Durchflusssteuerschicht 69 kann daher auch als Schaltschicht bezeichnet werden. Nach dem oben Gesagten dient die Durchflusssteuerschicht 69 zur Steuerung des Befüllvorgangs der Mikrokanäle 62 in dem Verdampfungskörper 60.

Die Durchflusssteuerschicht 69 kann vorteilhaft auch oder zusätzlich als EWOD (electro wetting on dielectrics)-Schicht ausgeführt sein, wobei die Oberfläche der Innenwand Durchgangsbohrungen 68 mit einem geeigneten Dielektrikum beschichtet ist. Ein solches Dielektrikum kann vorteilhaft als self assembled monolayer (SAM) ausgebildet sein, mit welchem der Kontaktwinkel zwischen Flüssigkeit und Innenwand einer Durchgangsbohrung 68 weiter oder zusätzlich beeinflusst werden kann.

Zwischen der Durchflusssteuerschicht 69 und dem Substrat 63 ist vorteilhaft eine Isolierschicht 70 aus einem isolierenden Material, beispielsweise Glas oder Keramik, mit sich durch die Isolierschicht 70 erstreckenden Durchgangsöffnungen 67 vorgesehen. Die Isolierschicht 70 dient dazu, den Verdampfungskörper 60 von dem Flüssigkeitsspeicher 18 thermisch zu isolieren, insbesondere um eine unerwünschte hohe Erwärmung und/oder eine Dampfbildung der Flüssigkeit in dem Flüssigkeitsspeicher 18 während der Verdampfung zu verhindern. Die Isolierschicht 70 kann auch zur elektrischen Isolierung des Substrats 63 von der Durchflusssteuerschicht 69 dienen, wodurch das Verdampfen und/oder Heizen von der Durchflusssteuerung entkoppelt werden kann. Die Durchgangsöffnungen 67 korrespondieren vorzugsweise mit den Mikrokanälen 62 und/oder mit den Durchgangsbohrungen 68, so dass durchgehende Mikrokanäle vom Flüssigkeitsspeicher 18 bis zu den Austrittsöffnungen 76 auf der Austrittsseite 64 des Substrats 63 geschaffen werden.

Die Mikrokanäle 62, Durchgangsöffnungen 67 und/oder Durchgangsbohrungen 68 sind vorzugsweise mit ihrer Längsachse quer zu den Schichten 69, 70 angeordnet. Verallgemeinert sind, wenn durch das Substrat 63 und die Durchflusssteuerschicht 69 und/oder die Isolierschicht 70 und/oder mindestens eine andere Schicht eine Schichtenfolge gebildet ist, die Mikrokanäle 62 mit ihren Längsachsen vorteilhaft quer zu der Schichtenfolge angeordnet. Auf diese Weise lassen sich ein optimaler Wärmeeintrag von dem Substrat 63 in die Mikrokanäle 62 realisieren und die Mikrokanäle 62 von Abdichtungsproblem weitgehend freihalten. Des Weiteren weisen mehrere oder sämtliche Schichten der Schichtenfolge vorteilhaft unterschiedliche oder gleiche Schichtdicken auf, und zwar weiter vorzugsweise insbesondere jeweils eine Schichtdicke von kleiner oder gleich 500 µm. Auf diese Weise lässt sich ein optimaler Wärmeeintrag von dem Substrat 63 in die Mikrokanäle 62 realisieren und die Mikrokanäle 62 von Abdichtungsproblem weitgehend freihalten. Der Verdampfungskörper 60 kann dabei vorteilhaft aus Teilstücken eines Wafers mit Dünnfilmschichttechnologie hergestellt werden, welcher eine gebräuchliche Schichtdicke aufweist.

Die Mikrokanäle 62, Durchgangsöffnungen 67 und/oder Durchgangsbohrungen 68 können gleiche oder voneinander verschiedene Durchtrittsquerschnitte aufweisen. Die Anzahl K1 der Mikrokanäle 62, die Anzahl K2 der Durchgangsöffnungen 67 und/oder die Anzahl K3 der Durchgangsbohrungen 68 können sich voneinander unterscheiden. Insbesondere kann K1 größer sein als K2 und/oder größer sein als K3. K2 kann größer sein als K3. Einer Gruppe von Mikrokanälen 62, insbesondere mit einer Gruppenanzahl G1 kleiner oder gleich K1, kann eine einzige Durchgangsöffnung 67 und/oder eine einzige Durchgangsbohrung 68 zugeordnet sein, welche mit ihrem Querschnitt dem Querschnitt der Gruppe von Mikrokanälen 62 angepasst ist, ihm insbesondere entspricht oder übersteigt. Auf diese Weise kann eine Gruppe von mehreren Mikrokanälen 62 mit einer geringeren Anzahl an Durchgangsöffnungen 67 wahlweise gesperrt und/oder freigegeben und eine einfachere Fertigung realisiert werden. Auf diese Weise kann eine Gruppe von mehreren Mikrokanälen 62 mit einer geringeren Anzahl an Durchgangsbohrungen 68 zum Versorgen mit Flüssigkeit und einer einfacheren Fertigung realisiert werden. Abweichend zu dem in Fig. 3 dargestellten Ausführungsbeispiel kann beispielsweise einer Gruppe mit einer Gruppenanzahl G1 von 3 bis 10 Mikrokanälen 62 eine gemeinsame Durchgangsöffnung 67 und/oder eine gemeinsame Durchgangsbohrung 68 zugeordnet sein. Die Durchgangsöffnung 67 und/oder die Durchgangsbohrung 68 übergreift dabei die 3 bis 10 Mikrokanäle 62.

Anstelle der Nutzung des electro wetting-Effekts können auch andere den Durchfluss steuernde Elemente in der Durchflusssteuerschicht 69 oder allgemeiner der Durchflusssteuereinrichtung 66 vorgesehen sein, beispielsweise eine oder mehrere Drosseln und/oder eine oder mehrere steuerbare (Mikro-)Ventile. Sofern die Kapillarität der Mikrokanäle 62, Durchgangsöffnungen 67 und/oder Durchgangsbohrungen 68 zur Förderung einer ausreichenden Flüssigkeitsmenge vom Flüssigkeitsspeicher 18 in den Verdampfungskörper 60 nicht ausreicht, können zusätzliche oder alternative Fördermechanismen vorgesehen sein, beispielsweise durch Druckbeaufschlagung, eine oder mehrere (Mikro-)Pumpen oder dergleichen.

Die Verdampfungseinheit 29 ist so eingestellt, dass eine vorteilhafte Flüssigkeitsmenge im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfungseinheit 29 hinsichtlich der Flüssigkeitsmenge pro Zug einstellbar sein.

Die Dosierer/Verdampfer-Kombination kann vorteilhaft so eingestellt sein, dass überwiegend Flüssigkeitstropfen mit einem Durchmesser im Bereich zwischen 0,05 µm und 5 µm, bevorzugt zwischen 0,1 µm und 3 µm entstehen. Tröpfchengrößen im Bereich zwischen 0,05 und 5 MMAD (mass median aerodynamic diameter, massenmedianer aerodynamischer Durchmesser), vorzugsweise zwischen 0,1 und 3 MMAD, weiter vorzugsweise zwischen 0,5 und 2 MMAD, noch weiter vorzugsweise zwischen 0,7 und 1,5 MMAD, beispielsweise um ca. 1 MMAD können optimal sein. MMAD entspricht einer EU-Norm und wird in µm spezifiziert.

Da die Spannungsquelle 74 der Durchflusssteuereinrichtung 66 und die Spannungsquelle 71 des Verdampfungskörpers 60 separat elektrisch mit der Steueranordnung 29 verbunden sind und separat voneinander angesteuert werden, ist eine vorteilhafte funktionale Trennung zwischen der Förderung / Dosierung einerseits und Verdampfung andererseits realisiert.

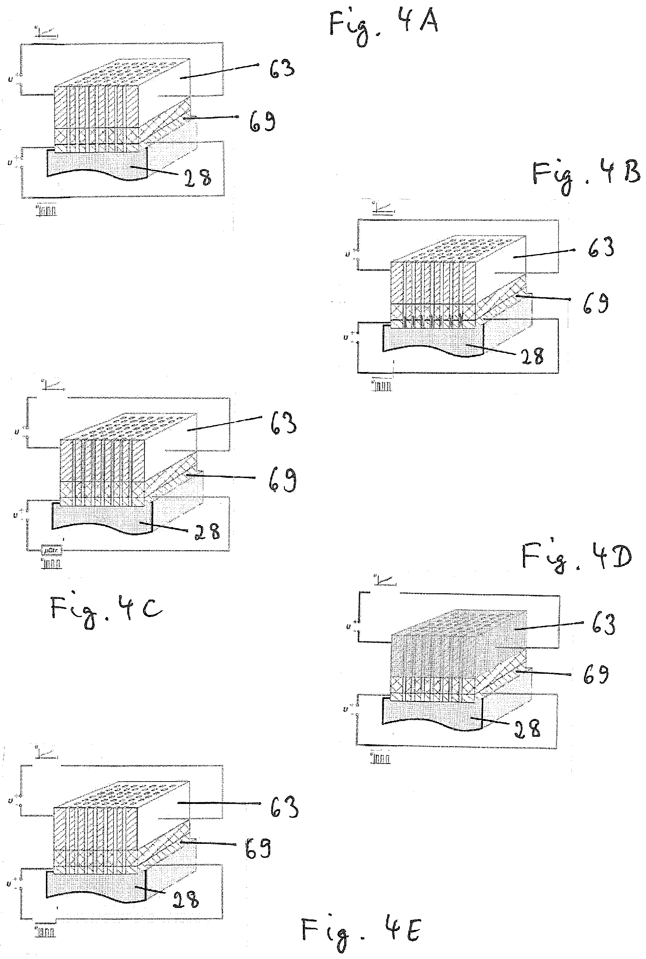

Der Ablauf des Verdampfungsvorgangs wird im Folgenden anhand der Figuren 4A bis 4E erläutert.

In dem in Figur 4A gezeigten Ausgangszustand ist die Spannungsquelle 74 für den Befüllvorgang spannungsfrei, der Kontaktwinkel zwischen Flüssigkeit und der Innenwand der Durchgangsbohrungen 68 ist hydrophob, so dass keine Flüssigkeit durch die Durchgangsbohrungen 68 gelangen kann.

Figur 4B illustriert den Befüllvorgang. Die Spannungsquelle 74 für den Befüllvorgang wird aktiviert, der Kontaktwinkel zwischen Flüssigkeit und der Innenwand der Durchgangsbohrungen 68 wird hydrophil, so dass Flüssigkeit kapillar durch die Durchgangsbohrungen 68 und die Durchgangsöffnungen in die Mikrokanäle 62 des Verdampfers 60 strömt.

Der Befüllvorgang ist abgeschlossen (Figur 4C), wenn alle Mikrokanäle 62 bis zur Auslassseite 64 des Verdampfers 60 befüllt sind. Im befüllten Zustand endet die Förderwirkung, da mit Erreichen der Auslassseite 64 keine kapillare Förderkraft mehr anliegt. Die Mikrokanäle 62 können durch gezieltes Ansteuern der Durchflusssteuerschicht 69 auch nur teilweise gefüllt sein.

Die jeweils zu verdampfende Menge kann somit über die Geometrie, insbesondere die Länge der Mikrokanäle 62 bzw. die Dicke des Substrats 63 und/oder durch Ansteuern der Durchflusssteuerschicht 69 vorgegeben werden. Generell ist die Länge der Mikrokanäle 62 groß im Verhältnis zu ihrem mittleren Durchmesser, insbesondere um mindestens einen Faktor drei größer, beispielsweise um einen Faktor im Bereich zwischen 3 und 30, vorzugsweise zwischen 5 und 20, beispielsweise um einen Faktor 10. Hierdurch ist die Erfindung abgrenzbar gegenüber bekannten Gittern oder Gitternetzen (sog. meshs).

Es ist denkbar, in einem bzw. unterschiedlichen Inhalatoren Verdampfungskörper 60 mit unterschiedlich dicken Substraten 63 zur Erzeugung von mehr oder weniger Dampf pro Verdampfungsvorgang / Zug einzusetzen bzw. anzusteuern.

Anschließend an den Befüllvorgang wird die Spannungsquelle 71 für den Verdampfungskörper 60 aktiviert (Heizvorgang, Figur 4D). Die Spannung Uh wird dabei so geführt, dass die Verdampfungstemperatur über das Substrat 63, d.h. in den Mikrokanälen 62, so eingestellt wird, dass sie an das individuelle Verdampfungsverhalten des eingesetzten Flüssigkeitsgemischs angepasst ist. Dies verhindert, auch wegen der großen Oberfläche der Kanalinnenwand zum Liquid, die Gefahr von lokaler Überhitzung und dadurch Schadstoffentstehung.

Sobald sämtliche Flüssigkeit verdampft ist, wird die Heizspannungsquelle 71 deaktiviert. Da die Liquideigenschaften und -menge vorteilhaft exakt bekannt sind, kann dieser Zeitpunkt sehr genau gesteuert werden. Die Energieaufnahme einer derartigen Verdampfereinheit 20 lässt sich daher gegenüber bekannten Verfahren reduzieren, da die benötigte Verdampfungsenergie viel dosierter und damit exakter eingebracht werden kann.

Da während des Verdampfungsvorgangs die Durchflusssteuerschicht 69 deaktiviert ist, kommt es nicht zu einer Rückwirkung mit dem restlichen Liquid im Flüssigkeitsspeicher 18, das heißt es tritt keine Konzentrationsänderung im Flüssigkeitsspeicher 18 auf. Die Verdampfung bzw. Wirkstoffdosierung ist deshalb über sämtliche Züge weitgehend identisch, da stets Liquid derselben Zusammensetzung verdampft wird.

Nach Abschluss des Heizvorgangs (Figur 4E) sind die Mikrokanäle 62 entleert. Ein möglicher verbleibender Liquidrest in den Durchgangsöffnungen 67 der Isolierschicht 70 kann konstruktiv über das Dickenverhältnis der Schichten 63, 69, 70 reduziert werden bzw. ist bezogen auf die je Zug verdampfte Liquidmenge vernachlässigbar. Somit kann mit dem nächsten Verdampfungsvorgang mit definierter, gleichbleibender Liquidzusammensetzung in Figur 4A wieder begonnen werden.

Die Spannungsquellen 71, 74 werden vorteilhaft mit einer geeigneten Ansteuerfrequenz typischerweise im Hz- oder kHz-Bereich und beispielsweise zwischen 1 Hz und 50 kHz, bevorzugt zwischen 30 Hz und 30 kHz, besonders bevorzugt zwischen 100 Hz und 25 kHz elektrisch angesteuert. In einer alternativen Ausgestaltung kann die Ansteuerfrequenz für die Spannungsquelle 74 im Bereich zwischen 5 Hz und 50 Hz, bevorzugt zwischen 10 Hz und 40 Hz liegen.

Die Verdampfereinheit 20 ist auf der Grundlage von MEMS-Technologie gefertigt und daher ein Mikro-Elektro-Mechanisches System.

## Patentansprüche

1. Verdampfereinheit (20) für einen Inhalator, mit einem Verdampfungskörper (60), welcher eine Einlassseite (61), eine Auslassseite (64), eine Mehrzahl von Mikrokanälen (62), ein Substrat (63) und ein Widerstandsheizelement zum Verdampfen von durch die Mikrokanäle (62) geförderter Flüssigkei aufweist, wobei
- die Mikrokanäle (62) sich jeweils von der Einlassseite (61) zu der Auslassseite (64) durch das Substrat (63) erstrecken,
- das Substrat (63) elektrisch leitfähig ist, und
- das Widerstandsheizelement von dem Substrat (63) gebildet ist, wobei die Mikrokanäle (62) jeweils von dem Substrat (63) in Umfangsrichtung umschlossen sind,
**dadurch gekennzeichnet, dass** die Verdampfereinheit (20) ein einheitliches Mikro-Elektro-Mechanisches System ist.

2. Verdampfereinheit nach Anspruch 1, **dadurch gekennzeichnet, dass** auf der Einlassseite (61) des Substrats (63) eine den Durchfluss von Flüssigkeit durch die Mikrokanäle (62) steuernde Durchflusssteuereinrichtung (66) vorgesehen ist.

3. Verdampfereinheit nach Anspruch 2, **dadurch gekennzeichnet, dass** die Durchflusssteuereinrichtung (66) eine Durchflusssteuerschicht (69) mit sich durch die Durchflusssteuerschicht (69) erstreckenden Durchgangsbohrungen (68) ist.

4. Verdampfereinheit nach Anspruch 3, **dadurch gekennzeichnet, dass** der Kontaktwinkel zwischen Flüssigkeit und der Innenwand einer oder jeder Durchgangsbohrung (68) durch Anlegen einer mittels einer Spannungsquelle erzeugbaren elektrischen Spannung veränderbar ist.

5. Verdampfereinheit nach Anspruch 3 oder 4, **dadurch gekennzeichnet, dass** zwischen der Durchflusssteuerschicht (69) und dem Substrat (63) eine Isolierschicht (70) mit sich durch die Isolierschicht (70) erstreckenden Durchgangsöffnungen (67) vorgesehen ist.

6. Verdampfereinheit nach einem der vorangehenden Ansprüche, **dadurch gekennzeichnet, dass** der mittlere Durchmesser eines oder jedes Mikrokanals (62) im Bereich zwischen 5 µm und 100 µm, vorzugsweise zwischen 10 µm und 50 µm, weiter vorzugsweise zwischen 20 µm und 40 µm liegt.

7. Verdampfereinheit nach einem der vorangehenden Ansprüche, **dadurch gekennzeichnet, dass** die Anzahl der Mikrokanäle (62) im Bereich zwischen 4 und 100 liegt.

8. Verdampfereinheit nach einem der vorangehenden Ansprüche, **dadurch gekennzeichnet, dass** die Mikrokanäle (62) in Form eines Arrays, insbesondere in Form von Spalten und Zeilen, angeordnet sind.

9. Verdampfereinheit nach Anspruch 8, **dadurch gekennzeichnet, dass** die Anzahl s der Spalten im Bereich zwischen 2 und 50, vorzugsweise zwischen 3 und 20, und/oder die Anzahl z der Zeilen im Bereich zwischen 2 und 50, vorzugsweise zwischen 3 und 20 liegt.

10. Verdampfereinheit nach einem der vorangehenden Ansprüche, **dadurch gekennzeichnet, dass** die Länge eines oder jedes Mikrokanals (62) im Bereich zwischen 100 µm und 500 µm, bevorzugt zwischen 150 µm und 400 µm, besonders bevorzugt zwischen 180 µm und 370 µm liegt.

11. Verdampfereinheit nach einem der vorangehenden Ansprüche, **dadurch gekennzeichnet, dass** die Länge eines oder jedes Mikrokanals (62) der Dicke des Substrats (63) entspricht.

12. Verdampfereinheit nach einem der vorangehenden Ansprüche, **dadurch gekennzeichnet, dass** der Abstand zwischen einander benachbarten Mikrokanälen (62) bezogen auf die Mittelachse einander benachbarter Mikrokanäle (62) im Bereich zwischen dem 1,3-fachen und dem 5-fachen, bevorzugt zwischen dem 1,5-fachen und dem 4,5-fachen, besonders bevorzugt im Bereich zwischen dem 2-fachen und dem 4-fachen des Durchmessers eines Mikrokanals (62) liegt.

13. Verdampfereinheit nach einem der Ansprüche 3 bis 5, **dadurch gekennzeichnet, dass** durch das Substrat (63) und die Durchflusssteuerschicht (69) und/oder die Isolierschicht (70) und/oder mindestens eine andere Schicht eine Schichtenfolge gebildet ist.

14. Verdampfereinheit nach Anspruch 13, **dadurch gekennzeichnet, dass** die Mikrokanäle (62) mit ihren Längsachsen quer zu der Schichtenfolge angeordnet sind.

15. Verdampfereinheit nach Anspruch 13 oder 14, **dadurch gekennzeichnet, dass** mehrere oder sämtliche Schichten der Schichtenfolge unterschiedliche oder gleiche Schichtdicken aufweisen, vorzugsweise insbesondere jeweils eine Schichtdicke von kleiner oder gleich 500 µm.

16. Verdampfereinheit nach einem der vorangehenden Ansprüche mit einem Datenspeicher (53; 59), **dadurch gekennzeichnet, dass** in dem Datenspeicher (53; 59) der Verdampfereinheit (20) eine dem verwendeten Flüssigkeitsgemisch angepasste Spannungskurve Uh(t) hinterlegt ist.

18. Verdampfereinheit nach einem der Ansprüche 3 bis 5, **dadurch gekennzeichnet, dass** sich die Anzahl K1 der Mikrokanäle (62), die Anzahl K2 der Durchgangsöffnungen (67) und/oder die Anzahl K3 der Durchgangsbohrungen (68) voneinander unterscheiden, und/oder einer Gruppe von Mikrokanälen (62), insbesondere mit einer Gruppenanzahl G1 kleiner oder gleich K1, eine einzige Durchgangsöffnung (67) und/oder eine einzige Durchgangsbohrung (68) zugeordnet ist.

19. Inhalator (10) mit einer Verdampfereinheit (20) nach einem der vorangehenden Ansprüche.

## Claims

1. Vaporizer unit (20) for an inhaler, having a vaporization body (60) which has an inlet side (61), an outlet side (64), a plurality of microchannels (62), a substrate (63) and has a resistance heating element for vaporizing liquid conveyed through the microchannels (62), wherein
- the microchannels (62) each extend through the substrate (63) from the inlet side (61) to the outlet side (64),
- the substrate (63) is electrically conductive, and
- the resistance heating element is formed by the substrate (63), wherein the microchannels (62) are each surrounded by the substrate (63) in a peripheral direction,
**characterized in that** the vaporizer unit (20) is a self-contained microelectromechanical system.

2. Vaporizer unit according to Claim 1, **characterized in that** a flow control device (66) controlling the flow of liquid through the microchannels (62) is provided on the inlet side (61) of the substrate (63).

3. Vaporizer unit according to Claim 2, **characterized in that** the flow control device (66) is a flow control layer (69) having passage bores (68) which extend through the flow control layer (69).

4. Vaporizer unit according to Claim 3, **characterized in that** the contact angle between the liquid and the inner wall of a or each passage bore (68) is able to be changed by applying an electrical voltage that is able to be generated by means of a voltage source.

5. Vaporizer unit according to Claim 3 or 4, **characterized in that** an insulating layer (70) having passage openings (67) which extend through the insulating layer (70) is provided between the flow control layer (69) and the substrate (63).

6. Vaporizer unit according to one of the preceding claims, **characterized in that** the average diameter of a or each microchannel (62) ranges between 5 µm and 100 µm, preferably between 10 µm and 50 µm, further preferably between 20 µm and 40 µm.

7. Vaporizer unit according to one of the preceding claims, **characterized in that** the number of microchannels (62) ranges between 4 and 100.

8. Vaporizer unit according to one of the preceding claims, **characterized in that** the microchannels (62) are arranged in the form of an array, in particular in the form of columns and rows.

9. Vaporizer unit according to Claim 8, **characterized in that** the number s of columns ranges between 2 and 50, preferably between 3 and 20, and/or the number z of rows ranges between 2 and 50, preferably between 3 and 20.

10. Vaporizer unit according to one of the preceding claims, **characterized in that** the length of a or each microchannel (62) ranges between 100 µm and 500 µm, preferably between 150 µm and 400 µm, particularly preferably between 180 µm and 370 µm.

11. Vaporizer unit according to one of the preceding claims, **characterized in that** the length of a or each microchannel (62) corresponds to the thickness of the substrate (63).

12. Vaporizer unit according to one of the preceding claims, **characterized in that** the distance between mutually adjacent microchannels (62) based on the central axes of mutually adjacent microchannels (62) ranges between 1.3 times and 5 times, preferably between 1.5 times and 4.5 times, particularly preferably between 2 times and 4 times, the diameter of a microchannel (62).

13. Vaporizer unit according to one of Claims 3 to 5, **characterized in that** a sequence of layers is formed by the substrate (63) and the flow control layer (69) and/or the insulating layer (70) and/or at least one other layer.

14. Vaporizer unit according to Claim 13, **characterized in that** the microchannels (62) are arranged with their longitudinal axes transverse to the sequence of layers.

15. Vaporizer unit according to Claim 13 or 14, **characterized in that** multiple layers or all the layers of the sequence of layers have different or equal layer thicknesses, preferably in particular each having a layer thickness of less than or equal to 500 µm.

16. Vaporizer unit according to one of the preceding claims, having a data memory (53; 59), **characterized in that** a voltage curve Uh(t) adapted to the liquid mixture used is stored in the data memory (53; 59) of the vaporizer unit (20).

18. Vaporizer unit according to one of Claims 3 to 5, **characterized in that** the number K1 of microchannels (62), the number K2 of passage openings (67) and/or the number K3 of passage bores (68) differ from one another, and/or a group of microchannels (62), in particular with a group number G1 less than or equal to K1, is assigned a single passage opening (67) and/or a single passage bore (68).

19. Inhaler (10) having an vaporizer unit (20) according to one of the preceding claims.

## Revendications

1. Unité d'évaporation (20) pour un inhalateur, avec un corps d'évaporation (60), qui comporte un côté d'entrée (61), un côté de sortie (64), une multitude de microcanaux (62), un substrat (63) et un élément chauffant à résistance pour évaporer un liquide transporté à travers les microcanaux (62),
- les microcanaux (62) s'étendant chacun à travers le substrat (63) depuis le côté d'entrée (61) au côté de sortie (64),
- le substrat (63) étant électriquement conducteur, et
- l'élément chauffant à résistance étant formé par le substrat (63), les microcanaux (62) étant entourés chacun par le substrat (63) dans la direction périphérique,
**caractérisée en ce que** l'unité d'évaporation (20) est un système micro-électromécanique uniforme.

2. Unité d'évaporation selon la revendication 1, **caractérisée en ce qu'**un dispositif de commande de débit (66) commandant le débit de liquide à travers les microcanaux (62) est prévu sur le côté d'entrée (61) du substrat (63).

3. Unité d'évaporation selon la revendication 2, **caractérisée en ce que** le dispositif de commande de débit (66) est une couche de commande de débit (69) avec des alésages traversants (68) s'étendant à travers la couche de commande de débit (69).

4. Unité d'évaporation selon la revendication 3, **caractérisée en ce que** l'angle de contact entre le liquide et la paroi intérieure d'un ou de chaque alésage traversant (68) peut varier par application d'une tension électrique pouvant être générée au moyen d'une source de tension.

5. Unité d'évaporation selon la revendication 3 ou 4, **caractérisée en ce qu'**une couche isolante (70) avec des ouvertures traversantes (67) s'étendant à travers la couche isolante (70) est prévue entre la couche de commande de débit (69) et le substrat (63).

6. Unité d'évaporation selon l'une des revendications précédentes, **caractérisée en ce que** le diamètre moyen d'un ou de chaque microcanal (62) se situe dans la plage entre 5 µm et 100 µm, de préférence entre 10 µm et 50 µm, mieux encore entre 20 µm et 40 µm.

7. Unité d'évaporation selon l'une des revendications précédentes, **caractérisée en ce que** le nombre des microcanaux (62) se situe dans la plage entre 4 et 100.

8. Unité d'évaporation selon l'une des revendications précédentes, **caractérisée en ce que** les microcanaux (62) sont disposés sous la forme d'un réseau, en particulier sous la forme de colonnes et de rangées.

9. Unité d'évaporation selon la revendication 8, **caractérisée en ce que** le nombre s des colonnes se situe dans la plage entre 2 et 50, de préférence entre 3 et 20, et/ou le nombre z des rangées se situe dans la plage entre 2 et 50, de préférence entre 3 et 20.

10. Unité d'évaporation selon l'une des revendications précédentes, **caractérisée en ce que** la longueur d'un ou de chaque microcanal (62) se situe dans la plage entre 100 µm et 500 µm, de manière préférée entre 150 µm et 400 µm, de manière particulièrement préférée entre 180 µm et 370 µm.

11. Unité d'évaporation selon l'une des revendications précédentes, **caractérisée en ce que** la longueur d'un ou de chaque microcanal (62) correspond à l'épaisseur du substrat (63).

12. Unité d'évaporation selon l'une des revendications précédentes, **caractérisée en ce que** la distance entre des microcanaux (62) mutuellement adjacents par rapport à l'axe central de microcanaux (62) mutuellement adjacents se situe dans la plage entre 1,3 fois et 5 fois, de manière préférée entre 1,5 fois et 4,5 fois, de manière particulièrement préférée dans la plage entre 2 fois et 4 fois le diamètre d'un microcanal (62).

13. Unité d'évaporation selon l'une des revendications 3 à 5, **caractérisée en ce qu'**une succession de couches est formée par le substrat (63) et la couche de commande de débit (69) et/ou la couche isolante (70) et/ou au moins une autre couche.

14. Unité d'évaporation selon la revendication 13, **caractérisée en ce que** les microcanaux (62) sont disposés avec leurs axes longitudinaux transversalement à la succession de couches.

15. Unité d'évaporation selon la revendication 13 ou 14, **caractérisée en ce que** plusieurs ou toutes les couches de la succession de couches présentent des épaisseurs de couche différentes ou identiques, de préférence en particulier chacune une épaisseur de couche inférieure ou égale à 500 µm.

16. Unité d'évaporation selon l'une des revendications précédentes, avec une mémoire de données (53 ; 59), **caractérisée en ce qu'**une courbe de tension Uh(t) adaptée au mélange liquide utilisé est enregistrée dans la mémoire de données (53 ; 59) de l'unité d'évaporation (20).

18. Unité d'évaporation selon l'une des revendications 3 à 5, **caractérisée en ce que** le nombre K1 des microcanaux (62), le nombre K2 des ouvertures traversantes (67) et/ou le nombre K3 des alésages traversants (68) diffèrent les uns des autres, et/ou une seule ouverture traversante (67) et/ou un seul alésage traversant (68) sont associés à un groupe de microcanaux (62), en particulier avec un nombre de groupes G1 inférieur ou égal à K1.

19. Inhalateur (10) avec une unité d'évaporation (20) selon l'une des revendications précédentes.
